# EUROPEAN PATENT SPECIFICATION

(11) **EP 0 995 771 B1**
(45) Date of publication and mention of the grant of the patent: **15.06.2005**
(21) Application number: 99308260.1
(22) Date of filing: 20.10.1999
(51) Int. Cl.: C08G 77/46

(54) **High purity oxyalkylene-modified organopolysiloxanes**
Hochreine Oxyalkylen-modifizierte Organopolysiloxane
Organopolysiloxanes de haute pureté contenant des motifs oxyalkylène

(30) Priority: 21.10.1998 US 176514
(43) Date of publication of application: 26.04.2000
(73) Proprietor: DOW CORNING CORPORATION, Midland Michigan 48686-0994 (US)
(72) Inventor: Petroff, Lenin James, Bay City, Michigan 48706 (US); Stanga, Michael Allen, Midland, Michigan 48642 (US); Rauscher, Wanda Wells, Angleton, Texas 77515 (US); Whitmarsh, Robert H., Lake Jackson, Texas 77566 (US); Upfield, Peter Cheshire, Trevaughan, Carmarthen SA33 6AA (GB)
(74) Representative: Kyle, Diana

(56) References cited:
- EP-A- 0 775 717
- US-A- 3 957 843
- US-A- 5 066 756
- PATENT ABSTRACTS OF JAPAN vol. 1996, no. 12, 26 December 1996 (1996-12-26) & JP 08 208426 A (NIPPON OIL &FATS CO LTD), 13 August 1996 (1996-08-13)

## Description

Our invention provides a method of making high purity oxyalkylene-modified organopolysiloxanes by reacting a mixture comprising a vinyloxy polyoxyalkylene compound, an organohydrogensiloxane compound and a platinum group metal-concaining catalyst. The resulting oxyalkylene-modified organopolysiloxanes are free from unwanted odor and do not contain excess polyoxyalkylene compound since the vinyloxy polyoxyalkylene compounds are not prone to isomerization during the reaction.

Current oxyalkylene-modified organopolysiloxane copolymers are prepared by reacting an organohydrogensiloxane and an olefinic polyoxyalkylene compound in the presence of a platinum-containing catalyst. Representative examples of these types of reactions are found in U.S. Patents 2,846,458, 3,842,112, 3,957,843 and 5,066,756.

Attempts have been made in the art to produce oxyalkylene-modified organopolysiloxanes which are free from unwanted odor and do not contain excess polyoxyalkylene compound. For example, U.S. Patent 5,118,764 discloses a method of producing a purified polyether silicone addition reaction product of a hydrogen siloxane and a polyether containing a terminal double bond. These unpurified reaction products contain one or both of unreacted polyether and internal rearrangement side reaction products which generate unpleasant odors upon storage and upon contact with water. Odor is reduced in these compositions by contacting the unpurified reaction products with an aqueous acidic solution until polyether and rearrangement products thereof are converted to odorous substances, and then subsequently removing the odorous substances from the thus-treated polyether silicone.

Japanese Patent Application Kokai 08208426 discloses a cosmetic base material which contains a modified silicone compound prepared by reaction of a hydrogen-concaining silicone compound with a polyoxyalkylene compound of the formula XO(AO)ₘY wherein X is a monovalent hydrocarbon group containing 5-30 carbon atoms having one double bond and no active hydrogen atom adjacent to the double bond, AO is an oxyalkylene containing 2-8 carbon atoms, optionally block or random, Y is hydrogen, a hydrocarbon group containing 1-24 carbon atoms or an acyl group containing 2-24 carbon atoms and m has a value of 1 to 1000. It further discloses that the materials do not cause odorization of modified silicone compounds.

European Patent Publication 0819719 discloses organopolysiloxanes containing -CH₂CH₂O(CR⁴R⁵)ₓOH groups wherein R⁴ and R⁵ may be che same or different groups at any given time and is selected from hydrogen or an alkyl group which can be branched or unbranched with up to a total of 12 carbon atoms and x has a value from 2 to 11, a process for preparing these organopolysiloxanes and their use as paint additives. An organopolysiloxane having the formula 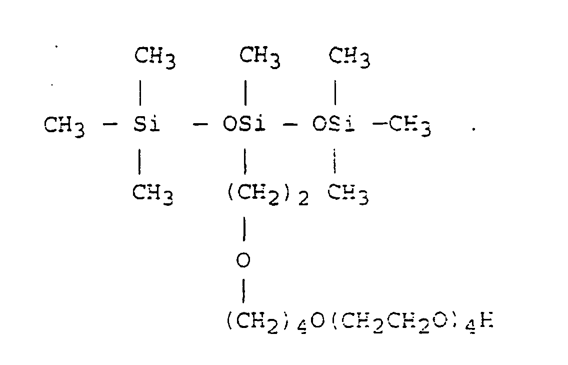 is disclosed along with a method of preparing these types of materials.

German Patent DE19631227 discloses cyclic siloxanes containing polyether groups which are useful as wetting agents or surfactants in aqueous preparations and as foam stabilizers in the manufacture of polyurethane foams.

In the reactions of the art, the allyl polyoxyalkylene compounds are known to isomerize to propenyl polyoxyalkylene compounds which is essentially non-reactive in a hydrosilylation reaction. As a result, a large excess of the allyl polyoxyalkylene compound is necessary in a formulation to compensate for the isomerization to ensure the reaction of all available SiH. The consequences of this unwanted isomerization reaction are several fold. First, the excess allyl polyoxyalkylene compound remains in the final product. As a result, the oxyalkylene-modified organopolysiloxane is diluted and not 100% active. Second, cost is added to the oxyalkylene-modified organopolysiloxane because excess reagent is needed. Third, the excess allyl polyoxyalkylene compound-is an unwanted impurity and can impact final product efficacy. Fourth, the excess allyl polyoxyalkylene compound is primarily responsible for unwanted odor in the product. The odor is formed as a result of the hydrolysis of the propenyl group forming propionaldehyde.

Our invention provides a method of making high purity cxyalkylene-modified organopolysiloxanes which comprises reacting a mixture comprising a vinyloxy polyoxyalkylene compound, an organohydrogensiloxane compound and a platinum group metal-containing catalyst.

Emulsions are also taught containing the oxyalkylene-modified organopolysiloxanes produced by the method of this invention.

It is an object of this invention to produce oxyalkylene-modified organopolysiloxanes which are free from unwanted odor and which do not contain excess polyoxyalkylene compounds.

Our invention provides oxyalkylene-modified organopolysiloxanes with starting materials which are not prone to isomerization during the reaction.

Our method of making high purity oxyalkylene-modified organopolysiloxanes comprises reacting a mixture comprising: (A) a vinyloxy polyoxyalkylene compound, (B) an organohydrogensiloxane compound and (C) a platinum group metal-containing catalyst.

"Reacting" for the purposes of this invention denotes simply mixing components (A) - (C) and any optional ingredients or heating a mixture of components (A)-(C) and any optional ingredients at temperatures above room temperature, preferably at temperatures above 50°C., and more preferably at temperatures from 50-150°C. Preferably, a mixture of components (A)-(C) and any optional ingredients is heated at temperatures from 50-150°C.

Component (A), the vinyloxy polyoxyalkylene compound is preferably a compound having its formula selected from CH₂=CHOR(OC₂H₄)ₐOR¹, CH₂=CHOR(OC₃H₆)_{b}OR¹, CH₂=CHOR (OC₄H₈)_{c}OR¹, CH₂=CHOR(OC₂H₄)ₐ(OC₃H₆)_{b}(OC₄H₈)_{c}OR¹, CH₂=CHOR(OC₂H₄)ₐ(OC₃H₆)_{b}OR¹, CH₂=CHOR(OC₂H₄)ₐ(OC₄H₈)_{c}OR¹ or CH₂=CHOR(OC₃H₆)_{b}(OC₄H₈)_{c}OR¹ wherein R is an alkylene group having at least two carbon atoms, R¹ is selected from a hydrogen atom, an alkyl group, an aryl group or an acyl group and a, b and c independently have an average value from 1 to 150.

The group R is an alkylene group having at least 2 carbon atoms which is exemplified by alkylene groups such as ethylene, butylene, 2-methyltrimechylene, hexamethylene, 3-ethyl-hexamethylene, octamethylene and (CH₂)₁₈-. Preferred alkylene groups have from 2 to 6 carbon atoms.

The group R¹ can be a.hydrogen atom, an alkyl group, an aryl group or an acyl group. The alkyl groups are exemplified by methyl, ethyl, propyl, butyl, hexyl, octyl and decyl. The aryl groups are exemplified by phenyl, tolyl and xylyl. The acyl group can have from 1 to 20 carbon atoms and include groups such as acetyl, propionyl, butyryl, isobutyryl, lauroyl, myristoyl and stearoyl 3-carboxypentadecanoyl. Preferably, the acyl group is a group having the formula -OCR⁴ wherein R⁴ denotes a monovalent hydrocarbon group. The monovalent hydrocarbon groups of R⁴ are exemplified by alkyl groups such as methyl, ethyl, propyl, butyl, hexyl, octyl and decyl, cycloaliphatic groups such as cyclohexyl, aryl groups such as phenyl, tolyl and xylyl and aralkyl groups such as benzyl and phenylethyl. It is preferred that R⁴ is an alkyl group such as methyl, ethyl or butyl.

In the above formula, preferably a, b and c independently have an average value from 1 to 50, especially from 1 to 25.

Preferably component (A) is selected from

CH₂=CHO(CH₂)ₙ(OC₂H₄)ₐOH, CH₂=CHO(CH₂)ₙ(OC₂H₄)ₐ(OC₃H₆)_{b}OH,

CH₂=CHO(CH₂)ₙ(OC₂H₄)ₐOCH₃,

CH₂=CHO(CH₂)ₙ(OC₂H₄)ₐ(OC₃H₆)_{b}OCH₃,

CH₂=CHO(CH₂)ₙ(OC₂H₄)ₐOC(O)CH₃

or

CH₂=CHO(CH₂)ₙ(OC₂H₄)ₐ(OC₃H₆)_{b}OC(O)CH₃

wherein n has a value of 2-6, a has an average value of 1 to 50 and b has an average value of 1 to 50.

The vinyloxy polyoxyalkylene compounds of component (A) are prepared by reacting a vinyloxy alcohol exemplified by 2-(vinyloxy)ethanol (ethylene glycol vinyl ether, i.e. CH₂=CHO(CH₂)₂OH), 1,4-butanediol monovinyl ether (CH₂=CHO(CH₂)₄OH) and 6-(vinyloxy)-1-hexanol(1,6-hexanediol monovinyl ether, i.e. CH₂=CHO(CH₂)₆OH) with an alkylene oxide exemplified by ethylene oxide or both ethylene oxide and propylene oxide in the presence of a base catalyst exemplified by alkali metal hydroxides such as potassium hydroxide or sodium hydroxide.

Component (A) in this invention is present at levels of 5 to 95 weight percent, and preferably from 30 to 70 weight percent of the total composition.

Component (B) in the method of this invention is an organohydrogensiloxane compound. Component (B) is preferably an organohydrogenpolysiloxane compound which is free of aliphatic unsaturation and contains two or more silicon atoms linked by divalent radicals, an average of from one to two silicon-bonded monovalent radicals per silicon atom and an average of at least one silicon-bonded hydrogen atom per compound. The organohydrogensiloxane compounds suitable as Component (B) can be linear, branched or resinous.

Component (B) is exemplified by bis(trimethylsiloxy)dimethyldisiloxane, 1,1,1,3,5,5,5-heptamethyltrisiloxane, hexamethyltrisiloxane, pentamethyldisiloxane, 1,1,3,3-tetramethyldisiloxane, dimethylhydrogensiloxy-terminated dimethylpolysiloxanes, dimethylhydrogensiloxy-terminated methylhydrogenpolysiloxanes, dimethylhydrogensiloxy-terminated dimethylpolysiloxane-methylhydrogenpolysiloxane copolymers, trimethylsiloxy-terminated dimethylpolysiloxane-methylhydrogenpolysiloxane copolymers, trimethylsiloxy-terminated methylhydrogenpolysiloxanes, an alkylhydrogensiloxane selected from R⁵Si(OSiMe₂H)₃, R⁵Si((OSiMe2)ₓOSiMe₂H)₃, (HMe₂SiO)₂-Si(R⁵)-O-(R⁵)Si-(OSiMe₂H)₂ or (HMe₂SiO(Me₂SiO)ₓ)₂-Si(R⁵)-O-(R⁵)Si-((OSiMe₂)ₓOSiMe₂H)₂ wherein Me hereinafter denotes methyl, R⁵ is a C2 to C18 straight-chain or branched-chain alkyl substituent and x has a value of 1 to 200 and siloxane resin copolymers consisting essentially of Me₂HSiO_{1/2} units and SiO₂ units. The group R⁵ is exemplified by ethyl, n-propyl, isopropyl, 2-methylpropyl, 2,2-dimethylpropyl, butyl, 2-methylbutyl, 2,2-dimethylbutyl, 2,3-dimethylbutyl, 2,2,3-trimethylbutyl, pentyl, 2-methylpentyl, 3-methylpentyl, 2,2-dimethylpentyl, 2,3-dimethylpentyl, 2,4-dimethylpentyl, 3,3-dimethylpentyl, 3-ethylpentyl, hexyl, 2-methylhexyl, 3-methylhexyl, 2-methylhexyl, heptyl, octyl, nonyl, decyl, undecyl, dodecyl, tridecyl, tetradecyl, pentadecyl, hexadecyl, heptadecyl and octadecyl. It is preferred that R⁵ is selected from ethyl, propyl, butyl or octyl.

The viscosity at 25°C. of Component (B) is generally from 0.65 to 4000 mm²/s, and is preferably from 0.8 to 2000 mm²/s (1 mm²/s = 1 centistoke).

The organohydrogenpolysiloxanes of Component (B) are well known in the art, many of these are available commercially.

Component (B) in this invention, is generally present at levels of 5 to 95 weight percent, and preferably from 30 to 70 weight percent of the total composition.

Component (C) in the method of this invention is any platinum group metal-containing catalyst which facilitates the reaction of silicon-bonded hydrogen atoms with silicon-bonded alkenyl radicals. By platinum group, it is meant herein ruthenium, rhodium, palladium, osmium, iridium and platinum.

The platinum group metal-containing catalyst is preferably a platinum-containing catalyst since they are the most widely used, readily available and provide a more favorable effect for the compositions of this invention in terms of improved reaction rates. Platinum-containing catalysts can be a compound or complex of platinum metal.

Preferred platinum-containing catalysts include chloroplatinic acid, alcohol modified chloroplatinic acids, complexes of olefins and chloroplatinic acid, complexes of chloroplatinic acid and divinyltetramethyldisiloxane, halogenated alkali metal platinum compounds exemplified by potassium hexachloroplatinate, potassium hexaiodoplatinate, sodium hexachloroplatinate hexahydrate, potassium tetrabromoplatinate, potassium tetrachloroplatinate and sodium tetrachloroplatinate hydrate, fine platinum particles adsorbed on carbon carriers and platinum black.

A particularly preferred platinum-containing catalyst component in the compositions of this invention is a form of chloroplatinic acid, either as the commonly available hexahydrate form or as the anhydrous form, as taught by U.S. Patent 2,823,218. Another particularly useful catalyst is the composition that is obtained by a method comprising reacting chloroplatinic acid with an aliphatically unsaturated organosilicon compound such as divinyltetramethyldisiloxane, as disclosed by U.S. Patent 3,419,593, because of its easy dispersibility in organosilicon systems.

The amount of platinum-containing catalyst component that is used is not narrewly limited providing there is a sufficient amount to accelerate a reaction between the organohydrogensiloxane and vinyloxy polyoxyalkylene compound. The exact necessary amount of this catalyst component will depend on the particular catalyst utilized. Preferably the catalyst is added at an amount of 0.5 to 10 parts for every one million parts of (A), and it is highly preferred that the amount is at 1 to 4 parts by weight of platinum for every one million parts by weight of (A).

The mixture in the method of this invention can further comprise an organic solvent exemplified by alcohols such as ethanol or isopropanol, aromatic hydrocarbons such as toluene or xylene, ethers such as dioxane and tetrahydrofuran (THF), aliphatic hydrocarbons, esters, ketones and chlorinated hydrocarbons.

The oxyalkylene-modified organopolysiloxanes of this invention are particularly useful in hair care formulations, skin care formulations, cosmetics, in preparing silicone emulsions and in polyurethane foams.

Thus this invention further relates to an organopolysiloxane emulsion comprising: (A) an oxyalkylene-modified organopolysiloxane compound having its formula selected from:

R₃SiO(R₂SiO)ₓ(RR¹SiO)_{y}SiR₃, R₃SiO(RR¹SiO)_{y}SiR₃,

R¹R₂SiO(R₂SiO)ₓ(RR¹SiO)_{y}SiR₂R¹, R¹R₂SiO(RR¹SiO)_{y}SiR₂R¹

and

R¹R₂SiO(R₂SiO)ₓSiR₂R¹, R⁵Si(OSiMe₂R¹)₃,

R⁵Si((OSiMe2)_{z}OSiMe₂R¹)₃.

(R¹Me₂SiO)₂-Si(R⁵)-O-(R⁵)Si-(OSiMe₂R¹)₂,

(R¹Me₂SiO(Me₂SiO)_{z})₂-Si(R⁵)-O-(R⁵)Si-((OSiMe₂)_{z}OSiMe₂R¹)₂

or siloxane resin copolymers consisting essentially of Me₂R¹SiO_{1/2} units and SiO₂ units wherein R is a monovalent hydrocarbon radical having from 1 to 20 carbon atoms,
R¹ is a polyoxyalkylene group selected from

-CH₂CH₂OR²(OC₂H₄)ₐOR³,

-CH₂CH₂OR²(OC₃H₆)_{b}OR³,

-CH₂CH₂OR²(OC₄H₈)_{c}OR³,

-CH₂CH₂OR²(OC₂H₄)ₐ(OC₃H₆)_{b}(OC₄H₈)_{c}OR³,

-CH₂CH₂OR²(OC₂H₄)ₐ(OC₃H₆)_{b}OR³,

-CH₂CH₂OR²(OC₂H₄)ₐ(OC₄H₈)_{c}OR³

or

-CH₂CH₂OR²(OC₃H₆)_{b}(OC₄H₈)_{c}OR³

wherein R² is an alkylene group having at least 2 carbon atoms, R³ is selected from a hydrogen atom, an alkyl group, an aryl group or an acyl group, R⁵ is a C2 to C18 straight-chain or branched-chain alkyl substituent, a, b and c independently have an average value from 1 to 150, x has an average value of 1 to 1000, y has an average value of 1 to 100 and z has a value of 1 to 200, (B) at least one surfactant, (C) at least one volatile methylsiloxane fluid and (D) water. The emulsions of this invention can further comprise an inorganic salt.

In the above formula, R is a monovalent hydrocarbon group having from 1 to 20 carbon atoms exemplified by alkyl groups such as methyl, ethyl, propyl, butyl, hexyl, octyl and decyl, cycloaliphatic groups such as cyclohexyl, aryl groups such as phenyl, tolyl and xylyl and aralkyl groups such as benzyl and phenylethyl. It is preferred that R is selected from methyl or phenyl. The several R radicals can be identical or different, as desired.

The group R² is an alkylene group having at least 2 carbon atoms which is exemplified by alkylene groups exemplified by ethylene, butylene, 2-methyltrimethylene, hexamethylene, 3-ethyl-hexamethylene, octamethylene and -(CH₂)₁₈-. Preferred alkylene groups have from 2 to 6 carbon atoms.

The group R³ can be a hydrogen atom, an alkyl group, an aryl group or an acyl group. The alkyl groups are exemplified by methyl, ethyl, propyl, butyl, hexyl, octyl and decyl. The aryl groups are exemplified by phenyl, tolyl and xylyl. The acyl group can have from 1 to 20 carbon atoms and include groups such as acetyl, propionyl, butyryl, isobutyryl, lauroyl, myristoyl and stearoyl 3-carboxypentadecanoyl. Preferably, the acyl group is a group having the formula -OCR⁴ wherein R⁴ denotes a monovalent hydrocarbon group. The monovalent hydrocarbon groups of R⁴ are as delineated above for R. It is preferred that R⁴ is a lower alkyl group such as methyl, ethyl or butyl.

The group R⁵ is exemplified by ethyl, n-propyl, isopropyl, 2-methylpropyl, 2,2-dimethylpropyl, butyl, 2-methylbutyl, 2,2-dimethylbutyl, 2,3-dimethylbutyl, 2,2,3-trimethylbutyl, pentyl, 2-methylpentyl, 3-methylpentyl, 2,2-dimethylpentyl, 2,3-dimethylpentyl, 2,4-dimethylpentyl, 3,3-dimethylpentyl, 3-ethylpentyl, hexyl, 2-methylhexyl, 3-methylhexyl, 2-methylhexyl, heptyl, octyl, nonyl, decyl, undecyl, dodecyl, tridecyl, tetradecyl, pentadecyl, hexadecyl, heptadecyl and octadecyl. It is preferred that R⁵ is selected from ethyl, propyl, butyl or octyl.

In the above formula, preferably x has an average value from 1 to 100, and especially from 10 to 100, y has an average value from 1 to 50, and especially from 1 to 10, a, b and c independently have an average value from 1 to 50, preferably from 1 to 25.

Preferably R¹ is a polyoxyalkylene group selected from -CH₂CH₂O(CH₂)ₙ(OC₂H₄)ₐOH,

-CH₂CH₂O(CH₂)ₙ(OC₂H₄)ₐ(OC₃H₆)_{b}OH,

-CH₂CH₂O(CH₂)ₙ(OC₂H₄)ₐOCH₃,

-CH₂CH₂O(CH₂)ₙ(OC₂H₄)ₐ(OC₃H₆)_{b}OCH₃,

-CH₂CH₂O(CH₂)ₙ(OC₂H₄)ₐOC(O)CH₃

or

-CH₂CH₂O(CH₂)ₙ(OC₂H₄)ₐ(OC₃H₆)_{b}OC(O)CH₃

wherein n has a value of 2-6 and a and b independently have an average value of from 1 to 25. It is especially preferred that n has a value of 4.

Component (A) should be present in the organopolysiloxane emulsion compositions in an amount from 0.5 weight percent (wt%) to 8 wt%, and preferably from 1 to 5 wt%, where wt% is based on the total weight of the emulsion.

Component (B) in the emulsions of this invention is at least one surfactant. The surfactant may be an anionic, cationic, nonionic or amphoteric surfactant. The surfactants may be employed separately or in combinations of two or more. Suitable surfactants for the preparation of stable aqueous emulsions are known in the art.

Examples of suitable anionic surfactants include alkali metal sulforicinates, sulfonated glyceryl esters of fatty acids such as sulfonated monoglycerides of coconut oil acids, salts of sulfonated monovalent alcohol esters such as sodium oleylisethianate, amides of amino sulfonic acids such as the sodium salt of oleyl methyl tauride, sulfonated products of fatty acids nitriles such as palmitonitrile sulfonate, sulfonated aromatic hydrocarbons such as sodium alpha-naphthalene monosulfonate, condensation products of naphthalene sulfonic acids with formaldehyde, sodium octahydroanthracene sulfonate, alkali metal alkyl sulfates such as ammonium lauryl sulfate or triethanol amine lauryl sulfate, ether sulfates having alkyl groups of 8 or more carbon atoms such as sodium lauryl ether sulfate or sodium alkyl aryl ether sulfates, alkylarylsulfonates having 1 or more alkyl groups of 8 or more carbon atoms, alkylbenzenesulfonic acids which are exemplified by hexylbenzenesulfonic acid, octylbenzenesulfonic acid, decylbenzenesulfonic acid, dodecylbenzenesulfonic-acid, cetylbenzenesulfonic acid and myristylbenzenesulfonic acid, salts of alkylbenzenesulfonic acids, sulfuric esters of polyoxyethylene alkyl ether including CH₃(CH₂)₆CH₂O(C₂H₄O)₂SO₃H, CH₃(CH₂)₇CH₂O(C₂H₄O)_{3.5}SO₃H, CH₃(CH₂)₈CH₂O(C₂H₄O)₈SO₃H, CH₃(CH₂)₁₉CH₂O(C₂H₄O)₄SO₃H and CH₃(CH₂)₁₀CH₂O(C₂H₄O)₆SO₃H, sodium salts, potassium salts and amine salts of alkylnaphthylsulfonic acid.

Examples of suitable cationic surfactants include various fatty acid amines and amides and their derivatives and the salts of the fatty acid amines and amides. Examples of aliphatic fatty acid amines include dodecylamine acetate, octadecylamine acetate and acetates of the amines of tallow fatty acids, homologues of aromatic amines having fatty acids such as dodecylanalin, fatty amides derived from aliphatic diamines such as undecylimidazoline, fatty amides derived from aliphatic diamines such as undecylimidazoline, fatty amides derived from disubstituted amines such as oleylaminodiethylamine, derivatives of ethylene diamine, quaternary ammonium compounds and their salts which are exemplified by tallow trimethyl ammonium chloride, dioctadecyldimethyl ammonium chloride, didodecyldimethyl ammonium chloride, dihexadecyl ammonium chloride, alkyltrimethylammonium hydroxides such as octyltrimethylammonium hydroxide, dodecyltrimethylammonium hydroxide or hexadecyltrimethylammonium hydroxide, dialkyldimethylammonium hydroxides such as octyldimethylammonium hydroxide, decyldimethylammonium hydroxide, didodecyldimethylammonium hydroxide, dioctadecyldimethylammonium hydroxide, tallow trimethylammonium hydroxide, coconut oil, trimethylammonium hydroxide, methylpolyoxyethylene cocoammonium chloride and dipalmityl hydroxyethylammonium methosulfate, amide derivatives of amino alcohols such as beta-hydroxylethyl-stearylamide and amine salts of long chain fatty acids.

Examples of suitable nonionic surfactants include polyoxyethylene alkyl ethers, polyoxyethylene alkylphenol ethers, polyoxyethylene lauryl ethers, polyoxyethylene sorbitan monoleates, polyoxyethylene alkyl esters, polyoxyethylene sorbitan alkyl esters, polyethylene glycol, polypropylene glycol, diethylene glycol, ethoxylated trimethylnonanols and polyoxyalkylene glycol modified polysiloxane surfactants.

Examples of the amphoteric surfactants that can be used include amino acid surfactants and betaine acid surfactants. Combinations of 2 or 3 types of nonionic surfactants, combinations of nonionic surfactants and anionic surfactants and combinations of nonionic surfactants and cationic surfactants can also be employed as component (B).

Preferred surfactants as component (B) include trimethylnonyl polyethylene glycol ethers and polyethylene glycol ether alcohols containinc linear alkyl groups having from 11 to 15 carbon atoms such as 2,6,8-trimethyl-4-nonyloxypolyethylene oxyethanol (6 EO) (sold as Tergitol®TMN-6 by OSi Specialties, A Witco Company, Endicott, NY), 2,6,8-trimethyl-4-nonyloxypolyethylene oxyethanol (10 EO) (sold as Tergitol TMN-10 by OSi Specialties, A Witco Company, Endicott, NY), alkylene-oxypolyethylene oxyethanol (C₁₁₋₁₅ secondary alkyl, 9 EO) (sold as Tergitol®15-S-9 by OSi Specialties, A Witco Company, Endicott, NY), alkylene-oxypolyethylene oxyethanol (C₁₁₋₁₅ secondary alkyl, 15 EO) (sold as Tergitol®15-S-15 by OSi Specialties, A Witco Company, Endicott, NY), octylphenoxy polyethoxy ethanols having varying amounts of ethylene oxide units such as octylphenoxy polyethoxy ethanol (40 EO) (sold as Triton® X405 by Rohm and Haas Company, Philadelphia, Pa.), nonionic ethoxylated tridecyl ethers available from Emery Industries, Mauldin, S.C. under the general tradename Trycol, alkali metal salts of dialkyl sulfosuccinates available from American Cyanamid Company, Wayne, N.J. under the general tradename Aerosol, polyethoxylated quaternary ammonium salts and ethylene oxide condensation products of the primary fatty amines, available from Armak Company, Chicago, I11. under the tradenames Ethoquad, Ethomeen or Arquad and polyoxyalkylene glycol modified polysiloxanes. These preferred surfactants may also be obtained from other suppliers under different tradenames.

Component (B) should be present in the organopolysiloxane emulsion compositions in an amount from 0.1 to 7 wt%, and preferably from 0.2 to 3 wt%, said wt% being based on the total weight of the emulsion.

Component (C) in the emulsions of this invention is at least one volatile methylsiloxane fluid. The volatile methylsiloxane fluid preferably comprises (CH₃)_{d}SiO_{(4-d)/2} units wherein d has an average value of 2 to 3. The volatile methylsiloxane preferably comprises siloxane units selected from the group consisting of (CH₃)₃SiO_{1/2}, (CH₃)₂SiO_{2/2}, CH₃SiO_{3/2} and SiO_{4/2} units. Preferably the volatile methyl siloxane fluid consists essentially of dimethylsiloxane ((CH₃)₂SiO_{2/2}) units, and optionally, trimethylsiloxane ((CH₃)₃SiO_{1/2}) units. Preferably volatile methylsiloxane fluid (C) is selected from (i) at least one cyclic siloxane having the formula -((CH₃)₂SiO_{2/2})_{d}-, (ii) at least one linear polydimethylsiloxane having the formula (CH₃)₃SiO((CH₃)₂SiO)ₑSi(CH₃)₃ or (iii) mixtures of (i) and (ii), wherein d is an integer of from 3 to 6 and e is an integer from 0 to 4. Particularly preferred as component (C) are (i) linear polydimethylsiloxanes having a viscosity of from 0.65 to 10 mm²/s, and preferably from 0.65 to 5 mm²/s, and (ii) a mixture of (a) a cyclic siloxane having the formula -((CH₃)₂SiO_{2/2})₄- and (b) a cyclic siloxane having the formula -((CH₃)₂SiO_{2/2})₅-. Preferably, a major portion of the cyclic siloxane mixture comprises the tetramer species (x = 4).

Component (C) is present in the organopolysiloxane emulsion in an amount from 20 to 80 wt%, and preferably from 25 to 40 wt%, said wt% being based on the total weight of the emulsion.

Water (D) forms the remainder of the emulsions of this invention and is present at a level of from 1 to 99 wt%, preferably from 30 to 70 wt%, said wt% being based on the total weight of the emulsion.

The emulsions of this invention can further comprise a silicone gum having a viscosity of at least 1 million mm²/s. The silicone gum is exemplified by trimethylsiloxy-terminated polydimethylsiloxane gums having a viscosity of at least 1 million mm²/s and hydroxy terminated polydimethylsiloxane gums having a viscosity of at least 1 million mm²/s. Preferably the silicone gum is a hydroxy-terminated polydimethylsiloxane gum having a viscosity of at least 1 million mm²/s.

The silicone gum, if present in the organopolysiloxane emulsion, is used in an amount from 0.5 to 5 parts by weight per 100 parts by weight of the emulsion.

The emulsions of this invention can also further comprise an inorganic salt exemplified by sodium chloride and ammonium chloride. The inorganic salt, if present in the organopolysiloxane emulsion compositions, is used in an amount of up to 2 parts by weight per 100 parts by weight of the emulsion.

Preparation of the organopolysiloxane emulsions can be carried out by any conventional technique and are generally separated into two types, mechanical means and emulsion polymerization means. Mechanical means typically involve homogenizing a mixture of a polydiorganosiloxane, one or more surfactants and water using milling machinery such as a colloid mill or a sonolator to obtain the desired droplet sizes. Emulsion polymerization methods for making emulsions utilize low viscosity polymer precursors such as monomers or reactive oligomers, which are immiscible in water, a surfactant to stabilize the polymer precursor droplet in water and a water soluble polymerization catalyst, such as quaternary ammonium hydroxides, which polymerize cyclopolysiloxanes in the presence of water. These include tallow trimethylammonium hydroxide, quaternary ammonium chlorides such as tallow trimethylammonium chloride, metal hydroxides such as sodium hydroxide, strong mineral acids, aliphatically substituted benzenesulfonic acids and aliphatic sulfonic acids. These components are added to water, the mixture is stirred and polymerization is allowed to advance until the reaction is complete or the desired degree of polymerization is obtained.

The organopolysiloxane emulsions of this invention may also be in the form of organopolysiloxane macroemulsions or organopolysiloxane microemulsions. The emulsions of this invention may also contain optional ingredients, for example, antifreeze additives, biocides, organic softeners, antistatic agents, preservatives, dyes and flame retardants. Preferred preservatives include Kathon® LX (5-chloro-2-methyl-4-isothiazolin-3-one from Rohm and Haas, Philadelphia, PA.19106), Giv-gard® DXN (6-acetoxy-2,4-dimethyl-m-dioxane from Givaudan Corp., Clifton NJ 07014), Tektamer® A.D. (from Calgon Corp., Pittsburgh, PA 152300), Nuosept® 91,95 (from-Huls America, Inc., Piscataway, NJ 08854), Germaben® (diazolidinyl urea and parabens from Sutton Laboratories, Chatham, NJ 07928), Proxel® (from ICI Americas Inc., Wilmington, DE 19897), methyl paraben, propyl paraben, sorbic acid, benzoic acid and lauricidin. The above optional components can be present in the organopolysiloxane emulsions up to 20 weight percent of the total composition, however, it is preferred that the optional components comprise up to 5 wt% of the total composition.

### Reference Example 1

A 7.6 liter jacketed, stirred, stainless steel reactor instrumented to continuously monitor temperature and pressure was thoroughly cleaned and dried. An initial charge of 952 g of 1,4-butanediol mono-vinyl ether with 1.09 g of solid sodium hydroxide (NaOH) was made at ambient temperature. The reactor was closed and oxygen eliminated by nitrogen pressure and purge. A total of seven cycles was used. A nitrogen atmosphere of 27.6 kPa was left in the reactor. The catalyzed alcohol was then heated to 125°C. While the reactor was being heated, 2745 g of ethylene oxide was loaded into a weigh cell connected to the reactor. When the temperature stabilized at 125°C. and pressure at 159 kPa, addition of the ethylene oxide was begun through a dip-tube reaching into the catalyzed alcohol. The ethylene oxide addition rate was monitored and controlled so that reactor pressure at no time exceeded 448 kPa. A total time of 680 minutes were required to add all of the ethylene oxide. The reactor and contents were maintained at 125°C. for an additional 570 minutes to insure complete reaction of the ethylene oxide such that the level of ethylene oxide was less than 10 ppm. The reactor was cooled and residual base removed by treating with 12 grams of magnesium silicate. The product of this reaction was a polyoxyalkylene compound having the average formula CH₂=CHO(CH₂)₄(OC₂H₄)₇OH having a hydroxy equivalent weight of 450 and a vinyl equivalent weight of 510.

### Reference Example 2

A 7.6 liter jacketed, stirred, stainless steel reactor instrumented to continuously monitor temperature and pressure was thoroughly cleaned and dried. An initial charge of 704 g of 1,4 butanediol mono-vinyl ether with 1.70 g of solid potassium hydroxide (KOH) was made at ambient temperature. The reactor was closed and oxygen eliminated by nitrogen pressure and purge. A total of seven cycles was used. A nitrogen atmosphere of 27.6 kPa was left in the reactor. The catalyzed alcohol was then heated to 125°C. While the reactor was being heated, 4760 g of ethylene oxide was loaded into a weigh cell connected to the reactor. When the temperature stabilized at 125°C. and pressure at 153 kPa, addition of the ethylene oxide was begun through a dip-tube reaching into the catalyzed alcohol. The ethylene oxide addition rate was monitored and controlled so that reactor pressure at no time exceeded 414 kPa. A total time of 890 minutes were required to add all of the ethylene oxide. The reactor and_contents were maintained at 125°C. for an additional 240 minutes to insure complete reaction of the ethylene oxide, such that the level of ethylene oxide was less than 10 ppm. The reactor was cooled and residual base removed by treating with 20 g of magnesium silicate. The product of this reaction was a polyoxyalkylene compound having the average formula CH₂=CHO(CH₂)₄(OC₂H₄)₁₈OH having a hydroxy equivalent weight of 450 and a vinyl equivalent weight of 510.

### Example 1

To a 500 ml 3-neck flask was added 139.28 g of the compound prepared in Reference Example 1. The flask was equipped with a thermometer attached to a thermowatch, an addition funnel, condenser and an air-powered stirrer. To the addition funnel was added 60.72 g of 1,1,1,3,5,5,5-heptamethyltrisiloxane. The resulting vinyl/SiH stoichiometry was 1:1. Approximately 5% by volume of the heptamethyltrisiloxane was added to the reaction flask. To the flask was then added 0.1g of sodium acetate. The mixture was heated to 75°C. and catalyzed with 45 microliters of a 0.1 molar solution of chloroplatinic acid hexahydrate in isopropyl alcohol (IPA). An exotherm to 86°C. was detected. The remaining heptamethyltrisiloxane was metered into the stirring reaction flask over a 30 minute period. The reaction was allowed to stir at 100°C. for 49 hrs. SiH was monitored in the examples via its FTIR absorbance at 2150 cm⁻¹. The final SiH in this example was 44 ppm. The resulting compound is reported in Table 1.

### Example 2

A 500 ml three-neck flask was equipped as described in Example 1. To the flask was added 71.62 g of the compound prepared in Reference Example 1 and 0.052 g of sodium acetate. To the addition funnel was added 28.38 g of 1,1,1,3,5,5,5-heptamethyltrisiloxane. The resulting vinyl/SiH stoichiometry was 1.1:1. Approximately 5% by volume of the total heptamethyltrisiloxane was fed into the reaction flask. The contents were heated to 70°C. To the flask were then added 0.141 g of a 3% solution of chloroplatinic acid hexahydrate in IPA. An exotherm to 78°C. was observed. The remaining heptamethyltrisiloxane was metered into the flask over an hour period. The contents were allowed to stir at 100°C. for 44 hrs. The final SiH was 13 ppm. The resulting compound is reported in Table 1.

### Example 3

A 500 ml three-neck flask was equipped as described in Example 1. To the flask was added 69.64 g of the compound prepared in Reference Example 1. No sodium acetate was added to the reaction flask. To the addition funnel was added 30.36 g of 1,1,1,3,5,5,5-heptamethyltrisiloxane. The resulting vinyl/SiH stoichiometry was 1:1, Approximately 5% by volume of the total heptamethyltrisiloxane was fed into the reaction flask. The contends were heated to 70°C. To the flask were then added 0.110 g of a 3.8% solution of chloroplatinic acid hexahydrate in IPA. The remaining heptamethyltrisiloxane was metered into the flask over a 1 hour period. The contents were allowed to stir at 100°C. and the SiH monitored by FTIR. The final SiH was found to be 78 ppm. The resulting compound is reported in Table 1.

### Example 4

A 250-ml flask was equipped as described in Example 1. To the flask was added 69.64 g of the compound prepared in Reference Example 1, 0.06 g of sodium acetate and 33.33 g of isopropanol. To the addition funnel was added 30.36 g of 1,1,1,3,5,5,5-heptamethyltrisiloxane. The resulting vinyl/SiH stoichiometry was 1:1. Approximately 5% by volume of the total heptamethyltrisiloxane was fed into the reaction flask. The contents were heated to 75°C. To the flask were then added 0.023 g of platinum catalyst solution containing 2.05% by weight of a complex of chloroplatinic acid and divinyltetramethyldisiloxane. The remaining heptamethyltrisiloxane was metered into the flask over a 1 hour period. The contents were allowed to stir at IPA reflux for 6 hrs. The final SiH was 31 ppm. The resulting compound is reported in Table 1.

### Example 5

A 250 ml flask was equipped as described in Example 1. To the flask was added 69.64 g of the compound prepared in Reference Example 1, 0.06 g of sodium acetate and 33.33 g of isopropanol. To the addition funnel was added 30.36 g of 1,1,1,3,5,5,5-heptamethyltrisiloxane. The resulting vinyl/SiH stoichiometry was 1:1. Approximately 5% by volume of the total heptamethyltrisiloxane was fed into the reaction flask. The contents were heated to 75°C. To the flask were then added 0.0010 g of potassium tetrachloroplatinate (K₂PtCl₄). The remaining heptamethyltrisiloxane was metered into the flask over a 1 hour period. The contents were allowed to stir at IPA reflux for 3 hours. The final SiH was measured by FTIR and found to be 5 ppm. The resulting compound is reported in Table 1.

### Example 6

A 250 ml flask was equipped as described in Example 1. To the flask was added 20.42 g of the compound prepared in Reference Example 1, 0.07 g of sodium acetate and 33.33 g of isopropanol. To the addition funnel was added 79.58 g of a trimethylsiloxy-terminated dimethylpolysiloxane-methylhydrogenpolysiloxane copolymer having the formula Me₃SiO(Me₂SiO)₅₀₄(MeHSiO)_{19.5}SiMe₃. The resulting vinyl/SiH stoichiometry was 1:1. Approximately 5% by volume of the total methylhydrogenpolysiloxane was fed into the reaction flask. The contents were heated to 75°C. To the flask were then added 0.021 g of a platinum catalyst solution containing 2.05% by weight of Pt(IV). The -remaining methylhydrogenpolysiloxane was metered into the flask over a 1 hour period. The contents were allowed to stir at IPA reflux for 16 hrs. The final SiH was measured by FTIR and found to be 41 ppm. The resulting compound is reported in Table 1.

Nuclear Magnetic Resonance Spectroscopy (NMR) was used to characterize the compounds prepared in the preceding examples. Both ²⁹Si and ¹³C NMR results are summarized in Table 1.

**Table 1**

| NMR Analysis (wt%) | | | | | | | | | | |
|---|---|---|---|---|---|---|---|---|---|---|
| Example | terminal vinyl polyoxyalkylene | isomerized vinyl | diol polyoxyalkylene | siloxane cyclics | silane | product | D(R) | D(R¹) | D(H) | D |
| | | | | | | | | | | |
| 1 | 30.2 | 0 | 11.8 | 0 | 6.3 | 56.2 | 0.47 | 0.37 | 0 | 0 |
| 4 | 14.4 | 0 | 11.8 | 0 | 6.3 | 67.5 | 0.84 | 0.06 | 0 | 0 |
| 5 | 12.7 | 0 | 11.6 | 0 | 2.7 | 73 | 0.88 | 0.06 | 0 | 0 |
| 6 | 6.2 | 0 | 0.4 | 4.1 | | 89.5 | 10 | 0.4 | 2.4 | 320 |

In Table 1 hereinabove:
terminal vinyl polyoxyalkylene denotes:

   CH₂=CHO(CH₂)₄(OC₂H₄)_{8.3}OH
diol polyoxyalkylene denotes: HO-(C₂H₄O)_{16.6}-H
Silane denotes: Me₃SiR¹
D(R) denotes: -(MeRSiO_{2/2})-
D(R¹) denotes: -(MeR¹SiO_{2/2})-
D(H) denotes: -(MeHSiO_{2/2})-
D denotes: -(Me₂SiO_{2/2})-
R denotes: the group -(CH₂)₂O(CH₂)₄(OC₂H₄)_{8.3}OH
R¹ denotes: -OH or -O(C₂H₄O)_{8.3}(CH₂)₄OCH=CH₂

The product was a compound having the average formula: Me3SiO(Me2SiO)x(MeRSiO)ySiMe3 wherein R is as defined above, x has a value of 0 and y has a value of about 1 in Examples 1, 4 and and x has a value of 320 and y has a value of 10 in Example 6.

### Comparison Example 1

A silicone polyether copolymer was produced by hydrosilylation of an allyl polyoxyalkylene compound having the formula CH₂=CHCH₂(OC₂H₄)_{7.7}OH with 1,1,1,3,5,5,5-heptamethyltrisiloxane. The product was produced by reacting a formulation containing a total of 68 wt% of the allyl polyether and 32 wt% of 1,1,1,3,5,5,5-heptamethyltrisiloxane. The resulting vinyl/SiH molar stoichiometry is 1.25/1. A 1 wc% solution of chloroplatinic acid in IPA is added to yield a solution containing 4 ppm platinum as a function of the total combined weight of the allyl polyether and siloxane fluid. The mixture also contained 500 ppm of sodium acetate. The silicone polyether copolymer is prepared by loading all of the allyl polyether into a reaction vessel, adding the sodium acetate along with 15 wt% of the total 1,1,1,3,5,5,5-heptamethyltrisiloxane. The mixture is heated to 85°C. under an inert nitrogen atmosphere. The chloroplatinic acid solution in IPA is then introduced to the mixture. The resulting exotherm is monitored and once subsided, the remaining 1,1,1,3,5,5,5-heptamethyltrisiloxane is metered into the mixture so as to keep the temperature below 100°C. Once the addition is complete, the mixture is allowed to remain agitated at 100°C. for 3 hours. Excess volatiles are then removed at reduced pressure at 100°C. for 1 hour. The produce is then cooled and collected. NMR analysis was conducted as described above. In this case, no evidence of residual terminal vinyl is seen. The excess vinyl is all seen in an isomerized form where the double bond has migrated to the beta position. A summary is shown in Table 2 below.

**Table 2**

| NMR Analysis (wt%) | | | | | | | | | | |
|---|---|---|---|---|---|---|---|---|---|---|
| Example | terminal vinyl polyoxyalkylene | isomerited vinyl | diol polyoxyalkylene | siloxanùe cyclics | silane | product | D(R²) | D(R³) | D(H) | D |
| Compar. Ex. 1 | 0 | 15.8 | 8.2 | 0 | 0 | 76.0 | 0.97 | 0.03 | 0 | 0 |

In Table 2 hereinabove:
terminal vinyl polyoxyalkylene denotes:

   CH₂=CHCH₂(OC₂H₄)_{7.7}OH
diol polyoxyalkylene denotes: HO-(C₂H₄O)_{15.4}-H
Silane denotes: Me₃SiR³
D(R²) denotes: -(MeR²SiO_{2/2})-
D(R³) denotes: -(MeR³SiO_{2/2})-
R² denotes the group: -(CH₂)₂(OC₂H₄)_{7.7}OH and
R³ denotes: -OH or -O(C₂H₄O)CHCH=CH₂₋.
D(H) and D are as defined above.

The product was a compound having the average formula: Me₃SiO(MeR²SiO)_{y}SiMe₃ wherein R² is as defined above and y has a value of 1.

### Example 7

The compound produced in Example 1 was evaluated for its utility in reducing the surface tension of water. Solutions of the compound of Example 1 in water were prepared at various concentrations. These solutions had their surface tension determined utilizing a Cahn Dynamic Contact Angle Analyzer. Receding contact angle measurements were used to determine the surface tension. The relationship between surface tension in milli Newtons/meter (mN/m) vs. the log of the concentration of the compound of Example 1 in water is shown in Table 3 below.

**Table 3**

| Surface Tension for Solutions of Example 1 in Water | | | | |
|---|---|---|---|---|
| | | | surface | |
| weight | molarity | | tension | |
| percent | mol/kg | log(wt%) | mN/m | |
| 9.67E-04 | 2.15E-05 | -4.015 | 65.34 | |
| 1.52E-03 | 3.38E-04 | -3.818 | 57.92 | |
| 2.74E-03 | 6.08E-04 | -3.562 | 53.65 | |
| 5.98E-03 | 1.33E-04 | -3.223 | 41.66 | |
| 1.13E-02 | 2.51E-04 | -2.947 | 39.68 | |
| 2.48E-02 | 5.52E-04 | -2.606 | 32.18 | |
| 5.46E-02 | 1.21E-03 | -2.263 | 22.46 | |
| 7.72E-02 | 1.71E-03 | -2.112 | 23.81 | |
| 1.91E-01 | 4.24E-03 | -1.719 | 22.33 | |
| 3.80E-02 | 5.44E-03 | -2.420 | 22.92 | |
| 7.52E-01 | 1.67E-02 | -1.124 | 20.88 | |
| 1.88E-00 | 4.19E-02 | -0.726 | 21.66 | - |

### Example 8

A water-in-oil silicone emulsion was prepared with the compound prepared in Example 6. The emulsion was prepared from a formulation containing 33.6 g of dimethylcyclosiloxanes, 24 g of blend of 85 wt% of dimethylcyclosiloxanes and 15 wt% of a hydroxy-terminated polydimethylsiloxane gum having a viscosity of over 1 million mm²/s and 2.4 g of the compound prepared in Example 6 in the oil phase and 176.4 g of deionized water, 2.4 g sodium chloride and 1.2 g of TWEENÔ™ 20 (a polyoxyethylene (20) monolaurate having an HLB of 16.7 marketed by ICI Americas, Inc, Wilmington, DE) in the aqueous phase. The emulsion is prepared by initially weighing the components of the aqueous phase into a 400 ml beaker and then mixing until homogeneous and smooth. Air entrapment is avoided. Then, the oil phase components are mixed in a 600 ml beaker and mixed unit homogeneous. Again air entrapment is avoided. A high speed rotary mixer is inserted into the beaker containing the oil phase such that the blade is just off the bottom of the beaker. A double blade configuration is used. They are 2.54 cm apart. The lower blade is 5.02 cm in diameter and the top blade is 6.53 cm in diameter.
Gradually the speed of the stirrer is increased until a mix speed of 1376 RPM is reached. The water phase is added to the mixing oil phase at a rate of 20 g/min. Once added, the emulsion is mixed for an additional 15 min and then poured into vials for aging testing. Three stability tests were run: room temperature, 50°C. oven age and freeze/thaw testing. After one month, the emulsion was still stable in both the room temperature and oven aged tests. The emulsion remained one phase after five freeze/thaw cycles (this involved keeping the sample in a -20°C. freezer overnight, then removed and allowed to thaw back to room temperature, then returned to the freezer for the subsequent cycle.

### Example 9

A second water-in-oil silicone emulsion was prepared with the compound prepared in Example 6. The emulsion was prepared from a formulation containing 69.6 g of dimethylcyclosiloxanes and 2.4 g of the compound of Example 6 in the oil phase and 164.4 g deionized water, 2.4 g sodium chloride and 1.2 g Tween™ 20 in the aqueous phase. The emulsion was then prepared according to the procedure of Example 8. This emulsion was then subjected to the three stability tests described in Example 8. After one month, the emulsion was stable in the room temperature test. The material stored in the 50°C. oven had some evidence of separation exhibited by a cream on the top of the formulation. The emulsion remained one phase after five freeze/thaw cycles.

### Example 10

A flask was equipped as described in Example 1. To the flask was added 0.025 moles of the compound prepared in Reference Example 1, 25 g of anhydrous toluene and 0.0025 g of potassium tetrachloroplatinate. The reaction mixture was heated to reflux and 0.025 moles of a dimethylhydrogensiloxy-terminated polydimethylsiloxane having a viscosity of 17 mPa·s (1mPa·s = 1 centipoise) at 25°C. and containing 1056 ppm of H was added over a two hour period. The reaction was monitored by Infra-Red analysis (ca. 2100cm⁻¹) and left for twelve hours, upon which the SiH content was less than 10 ppm. The toluene was removed under reduced pressure and the product filtered, using a filter aid, to yield a clear fluid. The NMR analysis of this product is shown in Table 4 below.

### Example 11

A flask was equipped as described in Example 1. To the flask was added 0.015 moles of the compound prepared in Reference Example 1, 30 g of anhydrous toluene and 0.0025 g of potassium tetrachloroplatinate. The reaction mixture was heated to reflux and 0.015 moles of a dimethylhydrogensiloxy-terminated polydimethylsiloxane having a viscosity of 67 mPa·s at 25°C. and containing 365 ppm of H was added over a two hour period. The reaction was monitored by Infra-Red analysis (ca. 2100cm⁻¹) and left for twelve hours, upon which the SiH content was less than 10 ppm. The toluene was removed under reduced pressure and the product filtered, using a filter aid, to yield a clear fluid. The NMR analysis of this product is shown in Table 4 below.

### Example 12

A flask was equipped as described in Example 1. To the flask was added 0.0071 moles of the compound prepared in Reference Example 1, 30 g of anhydrous toluene and 0.0016 g of potassium tetrachloroplatinate. The reaction mixture was heated to reflux and 0.0071 moles of a dimethylhydrogensiloxy-terminated polydimethylsiloxane having a viscosity of 166 mPa·s at 25°C. and containing 234 ppm of H was added over a two hour period. The reaction was monitored by Infra-Red analysis (ca. 2100cm⁻¹) and left for twelve hours, upon which the SiH content was less than 10 ppm. The toluene was removed under reduced pressure and the product filtered, using a filter aid, to yield a clear fluid. The NMR analysis of this product is shown in Table 4 below.

**Table 4**

| Example | % Residual CH=CH₂O- referenced to SiCH₂CH₂O by ¹H NMR. | M(H) | M(R⁴) | M(R⁵) | D |
|---|---|---|---|---|---|
| 10 | 2.8 | - | 1.0 | 0.04 | 12.99 |
| 11 | 1.6 | - | 1.0 | 0.02 | 31.01 |
| 12 | 3.5 | - | 1.0 | 0.03 | 57.05 |

In Table 4:
M(R⁴) denotes R⁴Me₂SiO_{1/2}-,
M(R⁵) denotes R⁵Me₂SiO_{1/2}-,
D denotes -(MeSiO_{2/2})-,
R⁴ denotes the group -(CH₂)₂O(CH₂)₄(O₂H₄)₇OH,
R⁵ denotes -OH or -O(C₂H₄O)₇(CH₂)₄OCH=CH₂,
M(H) denotes -(HMeSiO_{1/2})- units.

The product in Examples 10-12 was a compound having the average formula: R⁴Me₂SiO(Me₂SiO)ₓSiMe₂R⁴ wherein R⁴ is as defined above, x has a value of 13 in Example 10, 31 in Example 11 and 57 in Example 12.

### Comparison Example 2

A flask was equipped as described in Example #4. To the flask was added an 0.025 moles of an allyl polyoxyalkylene having the formula CH₂=CHCH₂(OC₂H₄)₇OH, 25 g of anhydrous toluene and 0.0025 g of potassium tetrachloroplatinate. The reaction mixture was heated to reflux and then 0.025 moles of a dimethylhydrogensiloxy-terminated polydimethylsiloxane polymer having a viscosity of 17 mPa·s at 25°C. and containing 1056 ppm H, was added over a two hour period. The reaction was monitored by Infra-Red analysis (ca. 2100cm⁻¹) and left for twelve hours. ²⁹Si NMR analysis showed that only 80% of the SiH had reacted due to isomerization of the allylpolyoxyalkylene to the propenyloxy isomer.

### Comparison Example 3

A flask was equipped as described in Example #4. To the flask was added an 0.030 moles of an allyl polyoxyalkylene having the formula CH₂=CHCH₂(OC₂H₄)₁₈OH, 25 g of anhydrous toluene and 0.0025 g of potassium tetrachloroplatinate. The reaction mixture was heated to reflux and then 0.025 moles of a dimethylhydrogensiloxy-terminated polydimethylsiloxane polymer having a viscosity of 17 mPa·s at 25°C. and containing 1056 ppm H, was added over a two hour period. The reaction was monitored by Infra-Red analysis (ca. 2100cm⁻¹) and left for 12 hours upon which the SiH content was less than 10 ppm. The toluene was removed under reduced pressure and the product filtered, using a filter aid, to yield a clear fluid. The excess polyoxyalkylene was present as the isomerized propenyloxy isomer which was confirmed by NMR.

### Example 13

A flask was equipped as described in Example #4. To the flask was added 0.025 moles of the compound prepared in Reference Example 2, 30 g of anhydrous isopropanol, 2.07 x 10⁻³ moles of sodium acetate and chloroplatinic acid in isopropanol (5 x 10⁻⁵ moles of Pt/mole SiH). The reaction mixture was heated to reflux and 0.025 moles of a dimethylhydrogensiloxy-terminated polydimethylsiloxane polymer having a viscosity of 17 mPa·s at 25°C. and containing 1056 ppm H was added ever a 1 hour period. The reaction was monitored by Infra-Red analysis (ca. 2100cm⁻¹) and left for 12 hours, upon which the SiH content was less than 10 ppm. The isopropanol was removed under reduced pressure and the product filtered using a filter aid, to yield a clear fluid.

### Example 14

A flask was_ equipped as described in Example #4. To the flask was added 0.0124 moles of the compound prepared in Reference Example 2, 30 g of anhydrous isopropanol, 2.07 x 10⁻³ moles of sodium acetate and chloroplatinic acid in isopropanol (5 x 10⁻⁵ moles of Pt/mole SiH). The reaction mixture was heated to reflux and 0.0124 moles of a dimethylhydrogensiloxy-terminated polydimethylsiloxane polymer having a viscosity of 67 mPa·s at 25°C. and containing 365 ppm H was added over a two hour period. The reaction was monitored by Infra-Red analysis (ca. 2100cm⁻¹) and left for 12 hours, upon which the SiH content was less than 10 ppm. The isopropanol was removed under reduced pressure and the product filtered, using a filter aid, to yield a clear fluid.

### Example 15

A flask was equipped as described in Example #4. To the flask was added: 7.03 x 10⁻³ moles of the compound prepared in Reference Example 2, 30 g of anhydrous isopropanol, 7.5 x 10⁻⁴ moles of sodium acetate and chloroplatinic acid in isopropanol (5 x 10⁻⁵ moles of Pt/mole SiH). The reaction mixture was heated to reflux and 7.03 x 10⁻³ moles of a dimethylhydrogensiloxy-terminated polydimethylsiloxane polymer having a viscosity of 166 mPa·s at 25°C. and containing 234 ppm H was added over a 2 hour period. The reaction was monitored by Infra-Red analysis (ca. 2100cm⁻¹) and left for 12 hours, upon which the SiH content was less than 10 ppm. The isopropanol was removed under reduced pressure and the product filtered, using a filter aid, to yield a clear fluid. The NMR analysis of the products produced in Examples 13-15 is shown in Table 5 below.

**Table 5**

| Example | % Residual CH=CH₂O- referenced to SiCH₂CH₂O by ¹H NMR. | M(H) | M(R⁶) | M(R⁷) | D |
|---|---|---|---|---|---|
| 13 | 1.2 | - | 1.0 | 0.05 | 10.87 |
| 14 | 1.9 | - | 1.0 | 0.02 | 37.77 |
| 15 | 2.7 | - | 1.0 | 0.02 | 65.39 |

In Table 5:
M(R⁶) denotes MeR⁶SiO_{1/2}-,
M(R⁷) denotes Me₂R⁷SiO_{1/2}-,
D denotes -(MeSiO_{2/2})-,
R⁶ denotes the group -(CH₂)₂O(CH₂)₄(OC₂H₄)₁₈OH,
R⁷ denotes -OH or -O(C₂H₄O)₁₈(CH₂)₄OCH=CH₂,
M(H) denotes -(Me₂HSiO_{1/2})- .

The product in Examples 13-15 was a compound having the average formula: R⁶Me₂SiO(Me₂SiO)ₓSiMe₂R⁶ wherein R⁶ is as defined above, x has a value of 11 in Example 13, 38 in Example 14 and 65 in Example 15.

### Example 16

A flask was equipped as described in Example #4. To the flask was added 0.046 moles of the compound produced in Reference Example 1, 30 g of anhydrous isopropanol, 4.55 x 10⁻³ moles of sodium acetate and chloroplatinic acid in isopropanol (5 x 10⁻⁵ moles Pt/mole SiH). The reaction mixture was heated to reflux and 0.046 moles of a trimethylsiloxy-terminated dimethylpolysiloxane-methylhydrogenpolysiloxane copolymer having a viscosity of 130 mPa·s at 25°C. and containing 1524 ppm H, was added over a 2 hour period. The reaction was monitored by Infra-Red analysis (ca. 2100cm⁻¹) and left for 12 hours, upon which the SiH content was less than 20 ppm. The isopropanol was removed under reduced pressure and the product filtered, using a filter aid, to yield a clear fluid.

### Example 17

A flask was equipped as described in Example #4. To the flask was added 0.023 moles of the compound produced in Reference Example 1, 30 g of anhydrous isopropanol, 2.27 x 10⁻³ moles of sodium acetate and chloroplatinic acid in isopropanol (5 x 10⁻⁵ moles Pt/mole SiH). The reaction mixture was heated to reflux and 0.023 moles of a trimethylsiloxy-terminated dimethylpolysiloxane-methylhydrogenpolysiloxane copolymer having a viscosity of 137 mPa·s at 25°C. and containing 796 ppm H, was added over a two hour period. The reaction was monitored by Infra-Red analysis (ca. 2100cm⁻¹) and left for twelve hours, upon which the SiH content was less than 20 ppm. The isopropanol was removed under reduced pressure and the product filtered, using a filter aid, to yield a clear fluid.

### Example 18

A flask was equipped as described in Example #4. To the flask was added 7.44 x 10⁻³ moles of the compound -produced in Reference Example 1, 30 g of anhydrous isopropanol, 6.8 x 10⁻⁴ moles of sodium acetate and chloroplatinic acid in isopropanol (5 x 10⁻⁵ moles Pt/mole SiH). The reaction mixture was heated to reflux and 7.44 x 10⁻³ moles of a trimethylsiloxy-terminated dimethylpolysiloxane-methylhydrogenpolysiloxane copolymer having a viscosity of 149 mPa·s at 25°C. and containing 248 ppm H, was added over a 2 hour period. The reaction was monitored by Infra-Red analysis (ca. 2100cm⁻¹) and left for 12 hours NMR analysis showed that the reaction had not gone to completion.

**Table 6**

| Example | % Residual CH=CH₂O- referenced to SiCH₂CH₂O by ¹H NMR. | D(H) | D(R⁸) | D(R⁹) | D |
|---|---|---|---|---|---|
| 16 | 11.6 | - | 3.12 | 0.35 | 40.03 |
| 17 | 9.2 | - | 2.19 | 0.20 | 46.61 |
| 18 | 16.1 | 0.06 | 0.56 | 0.05 | 49.09 |

In Table 6 hereinabove:
D(R⁸) denotes (MeR⁸SiO_{2/2})-
D(R⁹) denotes (MeR⁹SiO_{2/2})-
D(H) denotes -(MeHSiO_{2/2})-
D denotes: -(Me₂SiO_{2/2})-
R⁸ denotes the group -(CH₂)₂O(CH₂)₄(OC₂H₄)₇OH,
R⁹ denotes -OH or -O(C₂H₄O)₇(CH₂)₄OCH=CH₂,

The product was a compound having the average formula: Me₃SiO(Me₂SiO)ₓ(MeR⁸SiO)_{y}SiMe₃ wherein R⁸ is as defined above, x has a value of 40 and y has a value of 3 in Example 16, x has a value of 47 and y has a value of 2 in Example 17 and x has a value of 49 and y has a value of 1 in Example 18.

## Claims

1. A method of making oxyalkylene-modified organopolysiloxanes comprising reacting a mixture comprising:
(A) a vinyloxy polyoxyalkylene compound having its formula selected from
CH₂=CHOR(OC₂H₄)ₐOR¹ ,
CH₂=CHOR(OC₃H₆)_{b}OR¹ ,
CH₂=CHOR (OC₄H₈)_{c}OR¹ ,
CH₂=CHOR(OC₂H₄)ₐ(OC₃H₆)_{b}(OC₄H₈)_{c}OR¹ ,
CH₂=CHOR(OC₂H₄)ₐ(OC₃H₆)_{b}OR¹,
CH₂=CHOR(OC₂H₄)ₐ (OC₄H₈)_{c}OR¹
or
CH₂=CHOR(OC₃H₆)_{b}(OC₄H₈)_{c}OR¹
wherein R is an alkylene group having at least two carbon atoms, R¹ is selected from hydrogen atom, an alkyl group, an aryl group and an acyl group, and a, b and c independently have an average value from 1 to 150,
(B) an organohydrogensiloxane compound; and
(C) a platinum group metal-containing catalyst.

2. A method according to claim 1 wherein a, b and c independently have an.average value from 1 to 25.

3. A method according to claim 1 or 2, wherein (A) is selected from CH₂=CHO(CH₂)ₙ(OC₂H₄)ₐOH,
CH₂=CHO(CH₂)ₙ(OC₂H₄)ₐ(OC₃H₆)_{b}OH,
CH₂=CHO(CH₂)ₙ(OC₂H₄)ₐOCH₃,
CH₂=CHO(CH₂)ₙ(OC₂H₄)ₐ(OC₃H₆)_{b}OCH₃,
CH₂=CHO(CH₂)ₙ(OC₂H₄)ₐOC(O)CH₃
or
CH₂=CHO(CH₂)ₙ(OC₂H₄)ₐ(OC₃H₆)_{b}OC(O)CH₃
wherein n has a value of 2-6, a has an average value of 1 to 50 and b has an average value of 1 to 50.

4. A method according to any of claims 1 to 3, wherein (B) is selected from bis(trimethylsiloxy)dimethyldisiloxane, 1,1,1,3,5,5,5-heptamethyltrisiloxane, hexamethyltrisiloxane, pentamethyldisiloxane, 1,1,3,3-tetramethyldisiloxane, dimethylhydrogensiloxy-terminated dimethylpolysiloxanes, dimethylhydrogensiloxy-terminated methylhydrogenpolysiloxanes, dimethylhydrogensiloxy-terminated dimethylpolysiloxane-methylhydrogenpolysiloxane copolymers, trimethylsiloxy-terminated dimethylpolysiloxane-methylhydrogenpolysiloxane copolymers, trimethylsiloxy-terminated methylhydrogenpolysiloxanes, an alkylhydrogensiloxane selected from R⁵Si(OSiMe₂H)₃, R⁵Si((OSiMe2)ₓOSiMe₂H)₃, (HMe₂SiO)₂-Si(R⁵)-O-(R⁵)Si-(OSiMe₂H)₂ and (HMe₂SiO(Me₂SiO)ₓ)₂-Si(R⁵)-O-(R⁵)Si-((OSiMe₂)ₓOSiMe₂H)₂ wherein R⁵ is a C2 to C18 straight-chain or branched-chain alkyl substituent and x has a value of 1 to 200 and siloxane resin copolymers consisting essentially of Me₂HSiO_{1/2} units and SiO₂ units.

5. A method according to any of claims 1 to 4, wherein the mixture further comprises an organic solvent.

6. An organopolysiloxane emulsion comprising:
(A) an oxyalkylene-modified organopolysiloxane compound having its formula selected from:
R₃SiO(R₂SiO)ₓ(RR¹SiO)_{y}SiR₃,
R₃SiO(RR¹SiO)_{y}SiR₃,
R¹R₂SiO(R₂SiO)ₓ(RR¹SiO)_{y}SiR₂R¹,
R¹R₂SiO(RR¹SiO)_{y}SiR₂R¹,
R¹R₂SiO(R₂SiO)ₓSiR₂R¹
R⁵Si(OSiMe₂R¹)₃,
R⁵Si((OSiMe2)_{z}OSiMe₂R¹)₃,
(R¹Me₂SiO)₂-Si(R⁵)-O-(R⁵)Si-(OSiMe₂R¹)₂,
(R¹Me₂SiO(Me₂SiO)_{z})₂-Si(R⁵)-O-(R⁵)Si-((OSiMe₂)_{z}OSiMe₂R¹)₂
or siloxane resin copolymers consisting essentially of Me₂R¹SiO_{1/2} units and SiO₂ units wherein R is a monovalent hydrocarbon radical having from 1 to 20 carbon atoms, R¹ is a polyoxyalkylene group selected from
-CH₂CH₂OR²(OC₂H₄)ₐOR³,
-CH₂CH₂OR²(OC₃H₆)_{b}OR³,
-CH₂CH₂OR²(OC₄H₈)_{c}OR³,
-CH₂CH₂OR²(OC₂H₄)ₐ(OC₃H₆)_{b}(OC₄H₈)_{c}OR³,
-CH₂CH₂OR²(OC₂H₄)ₐ(OC₃H₆)_{b}OR³,
-CH₂CH₂OR²(OC₂H₄)ₐ(OC₄H₈)_{c}OR³
and
-CH₂CH₂OR²(OC₃H₆)_{b}(OC₄H₈)_{c}OR³
wherein R² is an alkylene group having at least 2 carbon atoms, R³ is selected from hydrogen atom, an alkyl group, an aryl group and an acyl group, R⁵ is a C2 to C18 straight-chain or branched-chain alkyl substituent, a, b and c independently have an average value from 1 to 150, x has an average value of 1 to 1000, y has an average value of 1 to 100 and z has a value of 1 to 200;
(B) at least one surfactant;
(C) at least one volatile methylsiloxane fluid; and
(D) water.

7. An emulsion according to claim 6 wherein R is methyl, R² is an alkylene group having from 2 to 6 carbon atoms, R³ is selected from hydrogen atom, methyl or an acyl group having the formula -OCR⁴ wherein R⁴ denotes an alkyl group, x has an average value from 10 to 100, y has an average value from 1 to 10 and a, b and c independently have an average value from 1 to 50.

8. An emulsion according to claim 6 or 7, wherein R¹ is a polyoxyalkylene group selected from
-CH₂CH₂O(CH₂)ₙ(OC₂H₄)ₐOH,
-CH₂CH₂O(CH₂)ₙ(OC₂H₄)ₐ(OC₃H₆)_{b}OH,
-CH₂CH₂O(CH₂)ₙ(OC₂H₄)ₐOCH₃,
-CH₂CH₂O(CH₂)ₙ(OC₂H₄)ₐ(OC₃H₆)_{b}OCH₃,
-CH₂CH₂O(CH₂)ₙ(OC₂H₄)ₐOC(O)CH₃
and
-CH₂CH₂O(CH₂)ₙ(OC₂H₄)ₐ(OC₃H₆)_{b}OC(O)CH₃
wherein n has a value of 2-6 and a and b independently have an average value of from 1 to 25.

9. An emulsion according to any of claims 6 to 8, wherein the emulsion further comprises a silicone gum having a viscosity of at least 1 million mm²/s.

10. An emulsion according to any of claims 6 to 9, wherein the emulsion further comprises an inorganic salt.

## Patentansprüche

1. Verfahren zur Herstellung von oxyalkylenmodifizierten Organopolysiloxanen, umfassend Umsetzen einer Mischung, die enthält:
(A) eine Vinyloxypolyoxyalkylenverbindung, deren Formel ausgewählt ist aus
CH₂=CHOR(OC₂H₄)ₐOR¹,
CH₂=CHOR(OC₃H₆)_{b}OR¹,
CH₂=CHOR(OC₄H₈)_{c}OR¹,
CH₂=CHOR(OC₂H₄)ₐ(OC₃H₆)_{b}(OC₄H₈)_{c}OR¹,
CH₂=CHOR(OC₂H₄)ₐ(OC₃H₆)_{b}OR¹,
CH₂=CHOR(OC₂H₄)ₐ(OC₄H₈)_{c}OR¹
oder
CH₂=CHOR(OC₃H₆)_{b}(OC₄H₈)_{c}OR¹,
worin R eine Alkylengruppe mit mindestens zwei Kohlenstoffatomen ist, R¹ aus einem Wasserstoffatom, einer Alkylgruppe, einer Arylgruppe und einer Acylgruppe ausgewählt ist und a, b und c unabhängig voneinander einen Mittelwert von 1 bis 150 haben,
(B) eine Organowasserstoffsiloxanverbindung und
(C) einen platingruppenmetallhaltigen Katalysator.

2. Verfahren nach Anspruch 1, wobei a, b und c unabhängig voneinander einen Mittelwert von 1 bis 25 haben.

3. Verfahren nach Anspruch 1 oder 2, wobei (A) ausgewählt ist aus
CH₂=CHO(CH₂)ₙ(OC₂H₄)ₐOH,
CH₂=CHO(CH₂)ₙ(OC₂H₄)ₐ(OC₃H₆)_{b}OH,
CH₂=CHO(CH₂)ₙ(OC₂H₄)ₐOCH₃,
CH₂=CHO(CH₂)ₙ(OC₂H₄)ₐ(OC₃H₆)_{b}OCH₃,
CH₂=CHO(CH₂)ₙ(OC₂H₄)ₐOC(O)CH₃
oder
CH₂=CHO(CH₂)ₙ(OC₂H₄)ₐ(OC₃H₆)_{b}OC(O)CH₃,
worin n einen Wert von 2-6 hat, a einen Mittelwert von 1 bis 50 hat und b einen Mittelwert von 1 bis 50 hat.

4. Verfahren nach einem der Ansprüche 1 bis 3, wobei (B) ausgewählt ist aus Bis(trimethylsiloxy)dimethyldisiloxan, 1,1,1,3,5,5,5-Heptamethyltrisiloxan, Hexamethyltrisiloxan, Pentamethyldisiloxan, 1,1,3,3-Tetramethyldisiloxan, dimethylwasserstoffsiloxyterminierten Dimethylpolysiloxanen, dimethylwasserstoffsiloxyterminierten Methylwasserstoffpolysiloxanen, dimethylwasserstoffsiloxyterminierten Dimethylpolysiloxan-Methylwasserstoffpolysiloxan-Copolymeren, trimethylsiloxyterminierten Dimethylpolysiloxan-Methylwasserstoffpolysiloxan-Copolymeren, trimethylsiloxyterminierten Methylwasserstoffpolysiloxanen, einem Alkylwasserstoffsiloxan, ausgewählt aus R⁵Si(OSiMe₂H)₃, R⁵Si((OSiMe₂)ₓOSiMe₂H)₃, (HMe₂SiO)₂-Si(R⁵)-O-(R⁵)Si-(OSiMe₂H)₂ und (HMe₂SiO(Me₂SiO)ₓ)₂-Si(R⁵)-O-(R⁵)Si-((OSiMe₂)ₓOSiMe₂H)₂, worin R⁵ ein geradkettiger oder verzweigtkettiger C₂-C₁₈-Alkylsubstituent ist und x einen Wert von 1 bis 200 hat, und Siloxanharzcopolymeren, die im Wesentlichen aus Me₂HSiO_{1/2}-Einheiten und SiO₂-Einheiten bestehen.

5. Verfahren nach einem der Ansprüche 1 bis 4, wobei die Mischung ferner ein organisches Lösungsmittel enthält.

6. Organopolysiloxanemulsion, enthaltend:
(A) eine oxyalkylenmodifizierte Organopolysiloxanverbindung, deren Formel ausgewählt ist aus:
R₃SiO(R₂SiO)ₓ(RR¹SiO)_{y}SiR₃,
R₃SiO(RR¹SiO)_{y}SiR₃,
R¹R₂SiO(R₂SiO)ₓ(RR¹SiO)_{y}SiR₂R¹,
R¹R₂SiO(RR¹SiO)_{y}SiR₂R¹,
R¹R₂SiO(R₂SiO)ₓSiR₂R¹,
R⁵Si(OSiMe₂R¹)₃,
R⁵Si((OSiMe₂)_{z}OSiMe₂R¹)₃,
(R¹Me₂SiO)₂-Si(R⁵)-O-(R⁵)Si-(OSiMe₂R¹)₂,
(R¹Me₂SiO(Me₂SiO)_{z})₂-Si(R⁵)-O-(R⁵)Si-((OSiMe₂)_{z}OSiMe₂R¹)₂
oder Siloxanharzcopolymeren, die im Wesentlichen aus Me₂R¹SiO_{1/2}-Einheiten und SiO₂-Einheiten bestehen, worin R ein monovalenter Kohlenwasserstoffrest mit 1 bis 20 Kohlenstoffatomen ist, R¹ eine Polyoxyalkylengruppe ist, ausgewählt aus
-CH₂CH₂OR²(OC₂H₄)ₐOR³,
-CH₂CH₂OR²(OC₃H₆)_{b}OR³,
-CH₂CH₂OR²(OC₄H₈)_{c}OR³,
-CH₂CH₂OR²(OC₂H₄)ₐ(OC₃H₆)_{b}(OC₄H₈)_{c}OR³,
-CH₂CH₂OR²(OC₂H₄)ₐ(OC₃H₆)_{b}OR³,
-CH₂CH₂OR²(OC₂H₄)ₐ(OC₄H₈)_{c}OR³
und
-CH₂CH₂OR²(OC₃H₆)_{b}(OC₄H₈)_{c}OR³,
worin R² eine Alkylengruppe mit mindestens zwei Kohlenstoffatomen ist, R³ aus einem Wasserstoffatom, einer Alkylgruppe, einer Arylgruppe und einer Acylgruppe ausgewählt ist, R⁵ ein geradkettiger oder verzweigtkettiger C₂-C₁₈-Alkylsubstituent ist, a, b und c unabhängig voneinander einen Mittelwert von 1 bis 150 haben, x einen Mittelwert von 1 bis 1000 hat, y einen Mittelwert von 1 bis 100 hat und z einen Wert von 1 bis 200 hat,
(B) mindestens eine oberflächenaktive Substanz,
(C) mindestens eine flüchtige Methylsiloxanflüssigkeit und
(D) Wasser.

7. Emulsion nach Anspruch 6, wobei R gleich Methyl ist, R² eine Alkylengruppe mit 2-6 Kohlenstoffatomen ist, R³ aus einem Wasserstoffatom, Methyl oder einer Acylgruppe mit der Formel -OCR⁴ ausgewählt ist, worin R⁴ eine Alkylgruppe bedeutet, x einen Mittelwert von 10 bis 100 hat, y einen Mittelwert von 1 bis 10 hat und ab, b und c unabhängig voneinander einen Mittelwert von 1 bis 50 haben.

8. Emulsion nach Anspruch 6 oder 7, wobei R¹ eine Polyoxyalkylengruppe ist, ausgewählt aus
-CH₂CH₂O(CH₂)ₙ(OC₂H₄)ₐOH,
-CH₂CH₂O(CH₂)ₙ(OC₂H₄)ₐ(OC₃H₆)_{b}OH,
-CH₂CH₂O(CH₂)ₙ(OC₂H₄)ₐOCH₃,
-CH₂CH₂O(CH₂)ₙ(OC₂H₄)ₐ(OC₃H₆)_{b}OCH₃,
-CH₂CH₂O(CH₂)ₙ(OC₂H₄)ₐOC(O)CH₃
und
-CH₂CH₂O(CH₂)ₙ(OC₂H₄)ₐ(OC₃H₆)_{b}OC(O)CH₃,
worin n einen Wert von 2-6 hat und a und b unabhängig voneinander einen Mittelwert von 1-25 haben.

9. Emulsion nach einem der Ansprüche 6 bis 8, wobei die Emulsion ferner ein Siliconharz mit einer Viskosität von mindestens 1 Million mm²/s enthält.

10. Emulsion nach einem der Ansprüche 6 bis 9, wobei die Emulsion ferner ein anorganisches Salz enthält.

## Revendications

1. Procédé de préparation d'organopolysiloxanes à modifications oxyalkylène comprenant l'étape consistant à faire réagir un mélange comprenant :
(A) un composé vinyloxypolyoxyalkylène ayant une formule choisie parmi
CH₂=CHOR(OC₂H₄)ₐOR¹,
CH₂=CHOR(OC₃H₆)_{b}OR¹,
CH²=CHOR(OC₄H₈)_{c}OR¹,
CH₂=CHOR(OC₂H₄)ₐ(OC₃H₆)_{b}(OC₄H₈)_{c}OR¹,
CH₂=CHOR(OC₂H₄)ₐ(OC₃H₆)_{b}OR¹,
CH₂=CHOR(OC₂H₄)ₐ(OC₄H₈)_{c}OR¹
ou
CH²=CHOR(OC₃H₆)_{b}(OC₄H₈)_{c}OR¹
dans lesquelles R représente un groupe alkylène ayant au moins deux atomes de carbone, R¹ est choisi parmi un atome d'hydrogène, un groupe alkyle, un groupe aryle ou un groupe acyle et a, b et c ont indépendamment une valeur moyenne de 1 à 150,
(A) un composé organohydrogènesiloxane ; et
(B) un catalyseur contenant un métal du groupe du platine.

2. Procédé selon la revendication 1 dans lequel a, b et c ont indépendamment une valeur moyenne de 1 à 25.

3. Procédé selon la revendication 1 ou 2, dans lequel (A) est choisi parmi
CH₂=CHO(CH₂)ₙ(OC₂H₄)ₐOH,
CH₂=CHO(CH₂)ₙ(OC₂H₄)ₐ(OC₃H₆)_{b}OH,
CH₂=CHO(CH₂)ₙ(OC₂H₄)ₐOCH₃,
CH₂=CHO(CH₂)ₙ(OC₂H₄)ₐ(OC₃H₆)_{b}OCH₃,
CH₂=CHO(CH₂)ₙ(OC₂H₄)ₐOC(O)CH₃
ou
CH₂=CHO(CH₂)ₙ(OC₂H₄)ₐ(OC₃H₆)_{b}OC(O)CH₃
dans lesquelles n a une valeur de 2 à 6, a a une valeur moyenne de 1 à 50 et b a une valeur moyenne de 1 à 50.

4. Procédé selon l'une quelconque des revendications 1 à 3, dans lequel (B) est choisi parmi le bis(triméthylsiloxy)diméthyldisiloxane, le 1,1,1,3,5,5,5-heptaméthyltrisiloxane, l'hexaméthyltrisiloxane, le pentaméthyldisiloxane, le 1,1,3,3-tétraméthyldisiloxane, les diméthylpolysiloxanes à groupe terminal diméthylhydrogènesiloxy, les méthylhydrogènepolysiloxanes à groupe terminal diméthylhydrogènesiloxy, les copolymères de diméthylpolysiloxane et de méthylhydrogènepolysiloxane à groupe terminal diméthylhydrogènesiloxy, les copolymères de diméthylpolysiloxane et de méthylhydrogènepolysiloxane à groupe terminal triméthylsiloxy, les méthylhydrogènepolysiloxanes à groupe terminal triméthylsiloxy, un alkylhydrogènesiloxane choisi parmi
R⁵Si(OSiMe₂H)₃,
R⁵Si((OSiMe2)ₓOSiMe₂H)₃,
(HMe₂SiO)₂-Si(R⁵)-O-(R⁵)Si-(OSiMe₂H)₂
et
(HMe₂SiO(Me₂SiO)ₓ)₂-Si(R⁵)-O- (R⁵)Si- ((OSiMe₂)ₓOSiMe₂H)₂
dans lesquelles R⁵ représente un substituant alkyle à chaîne linéaire ou à chaîne ramifiée en C2 à C18 et x a une valeur de 1 à 200 et des copolymères de résine de siloxane essentiellement composés d'unités Me₂HSiO_{1/2} et d'unités SiO₂.

5. Procédé selon l'une quelconque des revendications 1 à 4, dans lequel le mélange comprend en outre un solvant organique.

6. Emulsion d'organopolysiloxane comprenant :
(A) un composé d'organopolysiloxane à modifications oxyalkylène ayant une formule choisie parmi :
R₃SiO(R₂SiO)ₓ(RR¹SiO)_{y}SiR₃,
R₃SiO (RR¹SiO)_{y}SiR₃,
R¹R₂SiO(R₂SiO)ₓ(RR¹SiO)_{y}SiR₂R¹,
R¹R₂SiO(RR¹SiO)_{y}SiR₂R¹,
R¹R₂SiO(R₂SiO)ₓSiR₂R¹,
R⁵Si(OSiMe₂R¹)₃,
R⁵Si((OSiMe2)_{z}OSiMe₂R¹)₃,
(R¹Me₂SiO)₂-Si(R⁵)-O-(R⁵)Si- (OSiMe₂R¹)₂,
(R¹Me₂SiO(Me₂SiO)_{z})₂-Si(R⁵)-O-(R⁵)Si-((OSiMe₂)_{z}OSiMe₂R¹)₂
ou des copolymères de résine de siloxane essentiellement composés d'unités Me₂R¹SiO_{1/2} et d'unités SiO₂ dans lesquelles R représente un radical hydrocarbure monovalent ayant de 1 à 20 atomes de carbone, R¹ représente un groupe polyoxyalkylène choisi parmi
-CH₂CH₂OR²(OC₂H₄)ₐOR³,
-CH₂CH₂OR²(OC₃H₆)_{b}OR³,
-CH₂CH₂OR²(OC₄H₈)_{c}OR³,
-CH₂CH₂OR²(OC₂H₄)ₐ(OC₃H₆)_{b}(OC₄H₈)_{c}OR³,
-CH₂CH₂OR²(OC₂H₄)ₐ(OC₃H₆)_{b}OR³,
-CH₂CH₂OR²(OC₂H₄)ₐ(OC₄H₈)_{c}OR³
ou
-CH₂CH₂OR²(OC₃H₆)_{b}(OC₄H₈)_{c}OR³
dans lesquelles R² représente un groupe alkylène ayant au moins 2 atomes de carbone, R³ est choisi parmi un atome d'hydrogène, un groupe alkyle, un groupe aryle ou un groupe acyle, R⁵ représente un substituant alkyle à chaîne linéaire ou à chaîne ramifiée en C2 à C18, a, b et c ont indépendamment une valeur moyenne de 1 à 150, x a une valeur moyenne de 1 à 1000, y a une valeur moyenne de 1 à 100 et z a une valeur de 1 à 200 ;
(B) au moins un tensioactif ;
(C) au moins un fluide méthylsiloxane volatile ; et
(D) de l'eau.

7. Emulsion selon la revendication 6 dans laquelle R représente un groupe méthyle, R² représente un groupe alkylène ayant de 2 à 6 atomes de carbone, R³ est choisi parmi un atome d'hydrogène, un groupe méthyle ou acyle ayant la formule -OCR⁴ dans laquelle R⁴ désigne un groupe alkyle, x a une valeur moyenne de 10 à 100, y a une valeur moyenne de 1 à 10 et a, b et c ont indépendamment une valeur moyenne de 1 à 50.

8. Emulsion selon la revendication 6 ou 7, dans laquelle R¹ représente un groupe polyoxyalkylène choisi parmi
-CH₂CH₂O(CH₂)ₙ(OC₂H₄)ₐOH,
-CH₂CH₂O(CH₂)ₙ(OC₂H₄)ₐ(OC₃H₆)_{b}OH,
-CH₂CH₂O(CH₂)ₙ(OC₂H₄)ₐOCH₃,
-CH₂CH₂O(CH₂)ₙ(OC₂H₄)ₐ(OC₃H₆)_{b}OCH₃,
-CH₂CH₂O(CH₂)ₙ(OC₂H₄)ₐOC(O)CH₃
ou
-CH₂CH₂O(CH₂)ₙ(OC₂H₄)ₐ(OC₃H₆)_{b}OC(O)CH₃
dans lesquelles n a une valeur de 2 à 6 et a et b ont indépendamment une valeur moyenne de 1 à 25.

9. Emulsion selon l'une quelconque des revendications 6 à 8, dans laquelle l'émulsion comprend en outre une gomme de silicone ayant une viscosité d'au moins 1 million de mm²/s.

10. Emulsion selon l'une quelconque des revendications 6 à 9, dans laquelle l'émulsion comprend en outre un sel inorganique.
